# EUROPEAN PATENT SPECIFICATION

(11) **EP 3 730 180 B1**
(45) Date of publication and mention of the grant of the patent: **28.05.2025**
(21) Application number: 20173627.9
(22) Date of filing: 15.04.2010
(51) Int. Cl.: A61M 31/00, A61M 25/09, A61B 1/00, A61B 1/018, A61B 5/00

(54) **APPARATUS FOR ADVANCING A DEVICE FROM ONE BODY LUMEN TO ANOTHER**
VORRICHTUNG ZUR DURCHFÜHRUNG EINER VORRICHTUNG VON EINEM IN EIN ANDERES KÖRPERLUMEN
APPAREIL PERMETTANT DE FAIRE AVANCER UN DISPOSITIF D'UNE LUMIÈRE CORPORELLE À UN AUTRE

(30) Priority: 21.04.2009 US 171241
(43) Date of publication of application: 28.10.2020
(62) Divisional of application: 16160869.0
(73) Proprietor: Boston Scientific Scimed Inc., Maple Grove, Minnesota 55311-1566 (US)
(72) Inventor: PHAN, Hoang, Fremont, CA 94538 (US); LUNSFORD, John, San Carlos, CA 94070 (US); SANDER, Fiona, Los Altos Hills, CA 94024 (US); TO, Kevin, Campbell, CA 95008 (US); ALLEN, Michael, Los Altos, CA 94024 (US)
(74) Representative: Vossius & Partner Patentanwälte Rechtsanwälte mbB

(56) References cited:
- EP-A2- 1 247 500
- US-A- 5 275 611
- US-A1- 2004 225 304
- US-A1- 2006 210 605
- US-A1- 2009 030 380
- US-B2- 6 535 764

## Description

### BACKGROUND OF THE INVENTION

Field of the Invention. The present invention relates generally to medical apparatus. In particular, the present invention relates to a guidewire-like tissue penetrating device having the ability to hold adjacent tissue layers in apposition while tools are introduced therethrough.

A number of endoscopic and other intralumenal procedures require access from one body lumen into an adjacent body lumen. For example, a number of procedures may be performed by entering the gastrointestinal (GI) tract, particularly the esophagus, stomach, duodenum, small intestine, or large intestine, and passing tools from the GI tract into adjacent organs, ducts, cavities, and other structures, such as the bile duct, the pancreatic duct, the gallbladder, the urinary tract, a cyst or pseudocyst, an abscess, and the like. Such access into the adjacent body lumen will require forming a penetration or other access hole from within the first body lumen, through a wall of the first body lumen, through a wall of the second body lumen, and into the interior of the second body lumen. Moreover, depending on the procedure being performed, it will usually be necessary to place a catheter, a stent, a drainage tube, or the like through the penetrations that have been formed in each of the body lumens.

Of particular interest to the present invention, after a penetration has been formed from the first body lumen into a second body lumen, and a guidewire or other tracking device has been placed through such penetrations, it can be difficult to advance an interventional or other tool from the first body lumen, over the guidewire, into the second body lumen. It will be appreciated that most body lumens have relatively weak or flaccid wall structures. Many currently available interventional tools have atraumatic, blunt, or other distal ends which tend to push away the lumenal walls as they are engaged by the tool as the tool is advanced over the guidewire. This is particularly true of entry through the wall of the second body lumen into the interior thereof. Thus, even a successful placement of a guidewire from a first body lumen into a second body lumen does not guarantee successful introduction of a therapeutic or other device over the guidewire.

A particular problem can arise with translumenal penetration from the GI tract into an adjacent duct or lumen containing organ. Often, such access is necessary to place a catheter, stent, or other drainage device. Although the ductal structures such as the common bile duct and lumen containing organ such as the gall bladder are immediately adjacent to the stomach and small intestine, they are not attached and advancement of a guidewire or penetrating device into the gallbladder or bile duct from the stomach or small intestine can displace the target structure, resuling in leakage into the peritoneal cavity. Thus, it is desirable that lumenal apposition of the gallbladder or bile duct to the stomach or small intestine be achieved as soon as possible following first penetration and that it be maintained securely until the drainage catheter or stent can be placed.

For these reasons, it would be desirable to provide guidewires and other tracking devices which can be used to provide access from a first body lumen to a second body lumen in a manner which facilitates entry into the second body lumen. In particular, it would be desirable to provide guidewires and guidewire-like devices which can stabilize adjacent lumenal wall structures and prevent or inhibit leakage as a therapeutic or other tool is being introduced over the guidewire. Such tools and methods should be compatible with standard endoscopes and other sheaths which can be used to access a target location in the gastrointestinal tract or other body lumen. At least some of these objectives will be met by the inventions described hereinbelow.

Description of the Background Art. Guidewires and guidewire-like devices having inflatable occlusion balloons are described in U.S. Patent Nos. 4,790,813; 5,207,229; 5,209,727; 6,251,084; 6,475,185; 6,902,535; 6,942,678; 7,150,723; and 7,169,161. Trocars and trocar-like devices having balloons and other deployable anchors are described in U.S. Patent Nos. 3,039,468; 3,717,151; 4,608,965; 5,183,464; 5,197,971; 5,275,610; 5,290,249; 5,330,497; 5,353,785; 5,443,484; 5,688,247; 5,713,870; 5,817,062; 5,882,340; 5,935,107; 6,632,197; and 7,377,897. Other patents of interest include U.S. Patent Nos. 4,705,040; 5,275,611; 5,304,198; 6,080,174; 6,626,919; 6,635,068; and 7,331,980. US 6,535,764 discloses an anchor device for use with a stimulator. The anchor comprises an elongate body, an expandable distal portion having a sharp tip, a bipolar electrode pair, and a bumper located on a proximal portion of the elongate body. The expandable distal portion comprises a flexible disk for engaging the outer stomach wall. The disk has an inner surface that interfaces with the outer surface of stomach wall. US 2006/0210605 A1 relates to a balloon catheter for the treatment of paranasal sinuses.

### BRIEF SUMMARY OF THE INVENTION

The invention is defined in claim 1. Some embodiments are recited in the dependent claims. No method by itself is falling within the scope of the claims.

### Summary of the disclosure

The present disclosure provides methods and apparatus for establishing access tracts from a first body lumen to a second body lumen. In particular, the methods of the present disclosure will provide for apposition of the walls of the body lumen so that catheters, stents, and other tools can be advanced through the access tract with minimal or no leakage of the lumenal contents through the tract which is being formed. The apparatus comprises guidewires or guidewire-like devices having an anchor at or near their distal ends. The anchors may be deployed within the second or target body lumen so that the guidewire may be pulled or otherwise tensioned proximately to draw the wall of the second body lumen against the wall of the first body lumen. By maintaining a tension on the guidewire, the two walls may be maintained in close apposition in order to minimize or prevent leakage of the contents of both body lumens through the holes which have been formed in the lumenal walls. Such tissue apposition and stabilization also facilitates introduction of catheters and other tools over the guidewire or guidewire-like device while maintaining the tissue position and seal.

The anchors may take a variety of forms and will often be configured to provide a working space or a cavity on the interior side of the wall of the second body lumen. Such a working space or cavity allows advancement of a catheter, stent, or other working tool over the guidewire through the access tract and into the interior of the second body lumen without interference from the anchor. The apparatus may further include one or more deployable blade(s) for enlarging a tissue penetration as the guidewire is advanced. The guidewires may themselves have tissue-penetrating distal tips, in which case they can be used to form the initial penetration from the first body lumen to the second body lumen. Alternatively, the guidewires may have conventional "floppy" guidewire tips or other non-penetrating structures, where the guidewires may be introduced through a previously formed tissue tract formed by needles, trocars, or the like.

The methods and apparatus of the present disclosure may be used to form or to be passed through a preformed tissue access tract from any first body lumen to any adjacent second body lumen. Most commonly, the first body lumen will be part of the gastrointestinal (GI) tract including the esophagus, stomach, duodenum, small intestine, large intestine, and colon. The second body lumen will typically be a lumen or other cavity or structure which is adjacent to the gastrointestinal tract, including ducts such as the bile duct and the pancreatic duct, a lumen-containing organs such as the gallbladder and urinary bladder, solid tissue organs such as the pancreas, and liver, as well as diseased structures such as cysts, pseudocysts, abscesses, and the like. After access has been established, a variety of therapeutic or diagnostic tools may be introduced, typically by passing them coaxially over the guidewire in a conventional manner. The devices include catheters, stents, electrosurgical tools, drug delivery devices, implantable anchors, implantable pacing devices, and the like.

In a first aspect of the present disclosure, methods are provided for advancing a device from a first body lumen to a second body lumen. The methods comprise advancing a guidewire through the first body lumen to a target location. The guidewire is penetrated distally through a wall of the first body lumen at the target site and into the second body lumen through a wall of the second body lumen. Often, the guidewire will have a tissue-penetrating tip which allows the guidewire to form the first and second lumenal wall penetrations. Alternatively, the lumenal wall penetrations may have been previously formed using trocars, needles, or other tissue-penetrating devices. Initial placement of the guidewire will typically be achieved through an endoscope, sheath, or other tool which allows identification of the target location and steering or orientation of the guidewire toward the target location.

After the guidewire has been passed from the first body lumen, through the tissue tract and into the second body lumen, an anchor on the guidewire will be expanded within the second body lumen. By pulling the guidewire proximately, the expanded anchor may be engaged against the inner wall of the second body lumen to draw said wall against the wall of the first body lumen. The guidewire may continue to be pulled or may be fixed or immobilized in order to continue to apply tension to the guidewire and maintain the first and second lumenal walls in apposition. While the apposition is being maintained, a tool may be advanced over the tensioned guidewire from the first body lumen and into the second body lumen. Usually, tension will remain applied to the guidewire during the entire time the tool is being advanced. The first body lumen may be any body lumen, typically a lumen which is accessible through a natural body orifice, such as the gastrointestinal tract. The second body lumen will usually be an organ or other structure which is adjacent to the first body lumen, typically being one of the organs or structures listed above. The methods of the present disclosure will find particular use for accessing a pancreatic pseudocyst through the stomach or duodenum in order to place a stent or drainage catheter in order to drain the pseudocyst. The methods will also find use in accessing the bile and pancreatic ducts from the duodenum in order to drain the duct. The methods will find still further use in accessing the gallbladder from the duodenum or stomach in order to drain the gallbladder.

In most cases, the guidewire, or at least a portion thereof, will be sufficiently flexible so that it can be advanced through a sheath in the first body lumen and conform to the shape of the first body lumen. In other instances, however, the guidewire may be less flexible (more stiff), sometimes being substantially rigid, so that the first body lumen or other access passage will conform to the shape of the guidewire as it is being advanced.

In a preferred aspect of the present disclosure, expanding the anchor will comprise expanding an anchor having a concavity on a side adjacent to the wall of the second body lumen. The concavity allows a tool to be advanced so that its distal end is received within the concavity (i.e., the concavity provides a space that can accommodate the distal end of the tool as it advances through the tissue wall penetrations), without disrupting the anchor's deployment or its ability to continuously apply tension to the lumenal wall of the second body cavity. The anchors may have a variety of forms, including balloon anchors, mechanical elements, cage structures, or the like. The anchors may be symmetric so that they engage the second lumenal wall around an interface or lip which is generally circular and concentric with the access tract. In other cases, the anchor may be asymmetric so that it lies only on a single side of the access passage. In the latter cases, it may be possible to advance a catheter or other access tool over the guidewire with a distal end of the access tool passing on a portion of the guidewire where there is no anchor structure.

In certain exemplary embodiments of the present disclosure, the guidewire may include a deployable blade which may be opened prior to or during advancement of the guidewire through the tissue penetration. The deployable blade will enlarge the penetration which is being formed. Such an enlarged penetration can accommodate passage of larger therapeutic, diagnostic, or other tools. It will be appreciated, however, that an enlarged tissue penetration may require improved sealing while a treatment tool is being advanced and stabilized within the penetration.

In a second aspect of the present disclosure, a guidewire comprises a guidewire body having a distal end and a proximal end. An expansible anchor is disposed at or near the distal end of the guidewire body, and a blade is further disposed adjacent the expansible anchor. The blade will have a retracted configuration which conforms to the guidewire body and a deployed configuration having a cutting edge which extends outside of the guidewire body, Thus, advancement of the guidewire with the blade extended or deployed will enlarge the penetration by forming incision(s), usually aligned radially in the walls of the tissue tract in the tissue of the first and second body lumens. The construction and use of such cutting blades is described in detail in co-pending provisional application 61/171,228 (Attorney Docket No. 026923-001200US), the full disclosure of which is referred to.

The guidewire bodies will typically have a length in the range from 100 cm to 500 cm, more typically from 150 to 250 cm, and an outside'diameter, at least at the distal end, in the range from 0.4 mm to 5 mm, more usually from 0.5 mm to 2 mm. The guidewire body may have a solid core but will more typically have a hollow center in order to allow for inflation, expansion, or other manipulation of the anchor and optionally the blade(s). The anchor can take any of the forms discussed above, including balloons, cages, malecotts, self-deploying springs, flanges, cones, or the like. Similarly, a wide variety of different deployable blades may be provided. In its simplest form, the devices may include only a single blade mounted on a central pivot. Two, three, or four blades may alternatively be provided on individual pivots and may be deployed in a symmetric or asymmetric manner. Both the blades and the anchors may be self-deploying or alternatively may require separate deployment mechanisms in order to selectively expand and contract the anchors and/or blades. The blade will typically be disposed distally of the expansible anchor to allow the tissue tract to be enlarged prior to deployment and placement of the anchor. Alternatively, the blade and the anchor may be placed adjacent to each other, typically where the blade opens in a first radial orientation while the expansible anchor opens in a second radial orientation.

In a third aspect, the present disclosure comprises guidewires having a guidewire body with a distal end and a proximal end. An expansible anchor on the guidewire body has a retracted configuration which conforms to an external surface of the guidewire body and an expanded configuration that has a distal surface and a proximal surface. The proximal surface has a peripheral edge and a concave space or region within the peripheral edge. The concave space or region provides a working space when the peripheral edge of the anchor is drawn proximally against a tissue surface of the target body lumen. In particular, the working space allows a catheter, stent, or other tool or device to be advanced over the guidewire while the anchor continues to apply tension to maintain the first and second lumenal walls in apposition.

The particular dimensions of the catheter body are set forth above. The expansible anchor will typically have a peripheral diameter in the range from 1 mm to 20 mm and the cavity provided by the anchor will have a volume in the range from 0.05 ml to 1 ml. The cavity will usually have a generally conical shape with an apex attached to the catheter shaft and a peripheral base disposed generally concentrically about the catheter shaft at a location proximal to the apex. The guidewire may have either a tissue-penetrating tip, such as a sharpened tip, an electrosurgical tip, or the like. Alternatively or additionally, the distal tip of the guidewire body may be steerable.

### BRIEF DESCRIPTION OF THE DRAWINGS

Fig. 1 illustrates a guidewire having an expansible anchor constructed in accordance with the principles of the present disclosure
Fig. 2 illustrates a second embodiment of a guidewire having an expansible anchor and a deployable blade constructed in accordance with the principles of the present disclosure
Fig. 3 illustrates a balloon-type expansible anchor having a single inflation lumen with a valve to permit inflation and sealing of the balloon.
Fig. 4 illustrates a balloon-type expansible anchor having a working space or cavity on a proximal surface thereof.
Fig. 5 illustrates a guidewire having a distal cutting blade and a proximal, expansible anchor.
Fig. 6 illustrates a guidewire having an asymmetric expansible anchor and a catheter with an asymmetric end which can bypass the anchor when advanced over the guidewire.
Figs. 7A and 7B illustrate a low-profile anchor structure where a tubular guidewire body is split into three segments and may be deployed by axial foreshortening.
Fig. 8 illustrates a guidewire with an expansible braid anchor.
Fig. 9 illustrates a guidewire having a pair of self-deploying wire anchors and a deployable blade which is radially offset by 90° from the anchors.
Fig. 10 illustrates a guidewire having a deployable lumen anchor and radially offset deployable blade.
Fig. 11 illustrates a guidewire having a single structure which provides both a cutting blade and a deployable anchor.
Figs. 12A and 12B illustrate a guidewire having a segmented self-penetrating tip, where the tip may be axially advanced and deploy into three everting anchor elements.
Figs. 13A-13D illustrate a method in according to the present disclosure where a pair of adjacent tissue layers are held in apposition while a tool is advanced over a guidewire.

### DETAILED DESCRIPTION

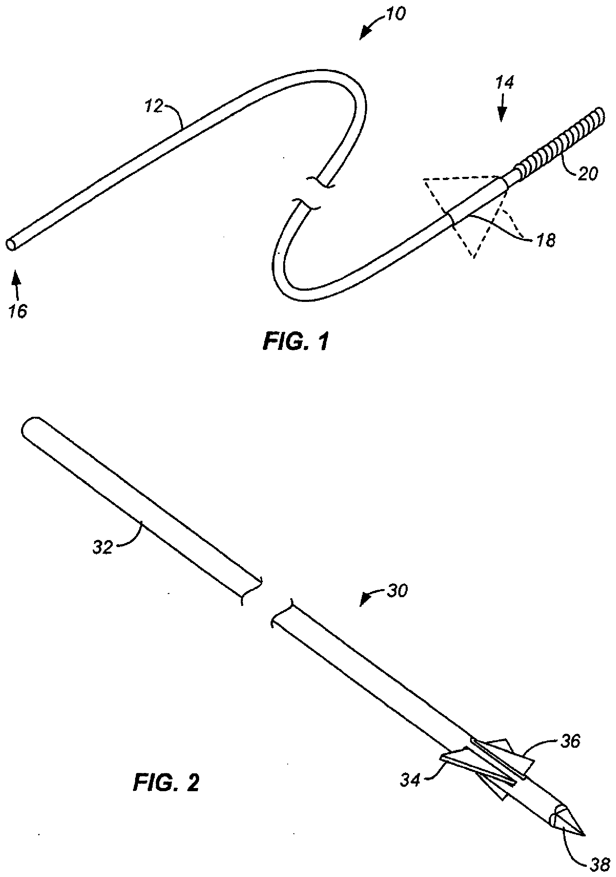

Referring to Fig. 1, a guidewire 10 constructed in accordance with the principles of the present disclosure comprises an elongate body 12 having a distal end 14 and a proximal end 16. An expansible anchor is disposed near the distal end 14 of the catheter body 12 and may be expanded from a contracted configuration (shown in full line) which conforms closely to the exterior of the catheter body to an expanded configuration (shown in broken line) which will have a width or diameter significantly greater than the diameter of the catheter body, typically being at least twice as wide, often being at least four times as wide, sometimes being six times as wide or greater. The expansible anchor 18 may have any one of a variety of structures and geometries. The expansible anchor 18 is shown as an inflatable balloon having a conical geometry with the wide portion or base oriented in a proximal direction. Thus, the base will be able to engage and seal against the tissue in the second or target body lumen when the guidewire is drawn in a proximal direction.

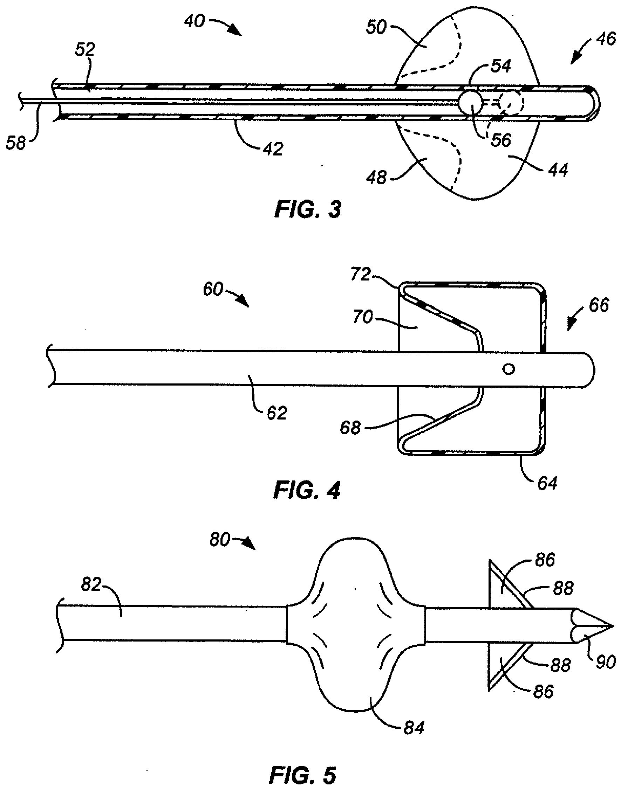

A first exemplary guidewire 10 may have the dimensions and properties generally associated with conventional medical guidewires. For example, the catheter body 12 may be sufficiently flexible to conform to a tortuous path through a body lumen as it is advanced. It may comprise a steerable tip 20 at its distal end to permit the catheter to be advanced and steered through a body lumen. It may be solid or hollow, typically being hollow to allow inflation of the balloon 18 (typically using a valving structure as described in more detail with respect to Fig. 3). Usually, it will be free of any structure at the proximal end which would prevent coaxial introduction of the catheter, stent, or other tool thereover. Alternatively, a removable hub or other structure (not shown) could be removably attached to the proximal end, for example for attaching to an inflation source for inflating the balloon.

A second exemplary guidewire structure 30 is illustrated in Fig. 2. Guidewire 30 includes a catheter body 32 which may be stiffer, optionally being rigid, in contrast to the guidewire body 12 of guidewire 10. For example, the guidewire body 32 may be formed from a relatively stiff hypotube over its entire length. Guidewire 30 is also illustrated to have both an anchor mechanism 34 and a blade mechanism 36, with particular deployment mechanisms for both the anchor and blades being illustrated hereinafter. Other blade actuation mechanisms are described in detail in co-pending application 61/171,228 (Attorney Docket No. 026923-001200US), the full disclosure of which is referred to. The guidewire 30 also includes a self-penetrating tip 38, which is illustrated as a faceted tip. Other sharpened tips and self-penetrating designs may be employed, including chamfered tips, electrosurgical tips, drilling tips, and the like.

Referring now to Fig. 3, a guidewire 40 comprises a guidewire body 42 having an inflatable balloon anchor 44 at a distal end 46 thereof. The inflatable balloon anchor 44 will preferably be formed from polyurethane, silicone or other elastomeric material so that advancement of a catheter or other tool over the guidewire body 42 allows the distal end of the tool to deform a proximal surface 48 of the balloon so that a recess or cavity 50 may be formed to accommodate the tool, as shown in broken line. As it is generally desirable to maintain a small diameter on the guidewire body 42, the guidewire 40 employs a single lumen 52 for inflation of the balloon. The balloon may be inflated by attaching a syringe or other inflation source to a proximal end (not shown) of the guidewire body 42 and closing the balloon inflation port 54 by drawing valve ball 56 proximally using valve wire 58 after the balloon has been inflated. In this way, the valve may be first open to permit inflation (as shown in broken line) and then closed to hold inflation within the balloon (as shown in solid line). It will be possible, of course, to place other valves within the single lumen 52. For example, a valve mechanism similar to a tire valve may be placed at or near the proximal end of the guidewire body 42. Inflation can' be introduced through the valve in a conventional manner (as for tires) and may be released by pressing the valve stem inward (again as is conventional with tire inflation mechanism). A wide variety of other single lumen balloon inflation and deflation mechanisms are known and described in the patent literature.

Referring now to Fig. 4, a guidewire 60 includes a guidewire body 62 having an inflatable balloon 64 at a distal end 66 thereof. The balloon 64 is generally cylindrical but has a pre-formed wall indentation 68 which defines a cavity or working space 70 when the balloon is fully inflated. Such a pre-shaped balloon may be formed from elastomeric materials (eg., silicone rubbers) but will more usually be formed from non-distensible materials such as polyethylene terephthalate, nylon, and the like). The cavity 70 is surrounded by a peripheral base or lip 72 which can engage a tissue wall surrounding a tissue penetration when the guidewire 60 is drawn proximally after it has been introduced into the body lumen and the balloon 64 has been inflated. The cavity 70 is useful to permit advancement of a catheter or other working tool through the tissue penetration or tract while the balloon 64 is being drawn against the tissue structure to apply tension to maintain apposition of adjacent tissue walls.

Referring now to Fig. 5, a guidewire 80 includes a guidewire body 82 having an inflatable balloon anchor 84 and a pair of deployable blades 86. Each blade has a forward cutting edge 88 which is disposed toward the tissue when the blade is fully deployed, as illustrated in Fig. 5. The inflatable balloon anchor 84 may be inflated by a single internal lumen, as previously described, and the blades 86 may be shifted between a constrained configuration (where they are fully retracted within the catheter body 82) and the deployed configuration which is illustrated. A wide variety of specific deployment configurations are illustrated in co-pending provisional application 61/171,228 (Attorney Docket No. 026923-001200US), which is referred to. The catheter 80 is shown with a sharpened, tissue-penetrating tip 90, so the guidewire can be used to form the penetration, enlarge the penetration with the deployable blades 88, and then seal the resulting enlarged tissue penetration with the balloon 84.

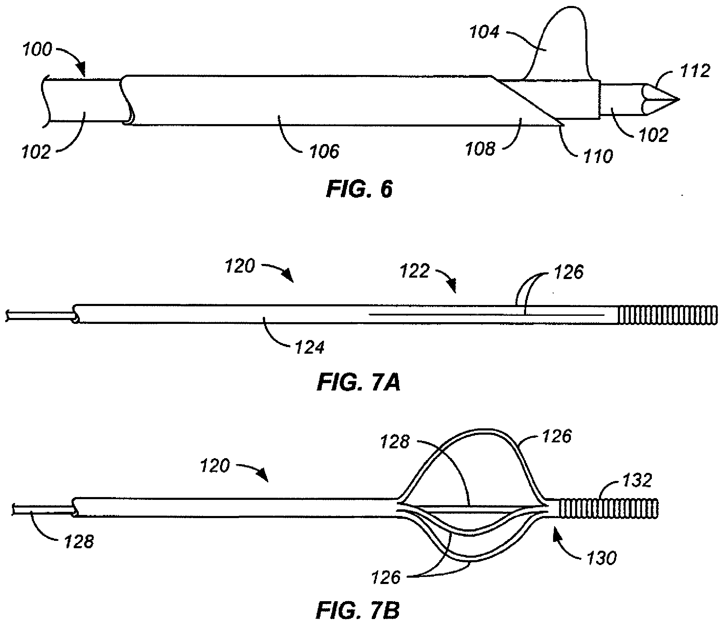

Referring now to Fig. 6, yet another guidewire 100 comprising a guidewire body 102 includes an asymmetric balloon anchor 104. The balloon anchor 104 extends outwardly from the guidewire body 102 in only one radial direction, leaving other radial portions of the guidewire free from structure. Thus, a catheter 106 having an angled end 108 may be advanced over the guidewire 100 and at least partially past the deployable balloon anchor 104 by orienting the leading tip 110 of the catheter so that it passes on a side of the guidewire opposite to that of the asymmetric balloon (the side free from anchor structure). The guidewire 100 is shown having self-penetrating tip 112 but could also be configured to have a blunt, steerable, or other tip.

Referring now to Figs. 7A and 7B, guidewire 120 may be constructed to have a particularly small diameter (low profile) by forming an expansible anchor structure 122 integrally within the guidewire body 124. The guidewire 124 comprises a hypotube or other small tubular structure. A distal portion of the tubular structure is split along three lines to form three separate elements 126 formed from an elastic material, typically an elastic material such as nitinol or stainless spring steel or alternatively an elastic polymer. The elements 126 are formed to lie in the radially constrained construction of Fig. 7A and may be radially expanded, as shown in Fig. 7B, by axially foreshortening the end of the guidewire body 124, typically by pulling on a tensioning member 128 to draw the distal end 130 in a proximal direction. The guidewire 120 is shown to have a steerable tip 132 but could also have a tissue-penetrating tip.

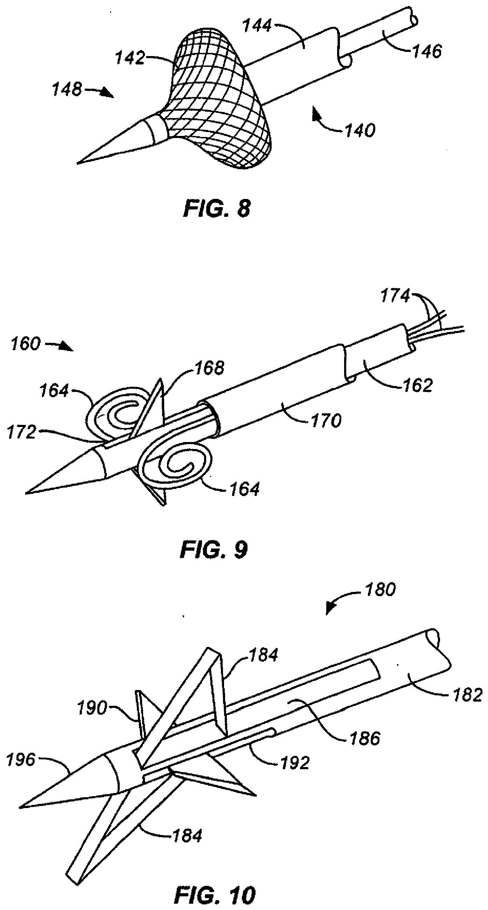

Referring now to Fig. 8, a guidewire 140 has an expansible anchor 142 in the form of a radially expanding braid. The braid will be formed as a tubular structure having a diameter similar to that of the guidewire body 144. By drawing on a central member 146, the distal tip 148 of the guidewire may be foreshortened to radially expand the braid 142, as shown in Fig. 8. The construction of suitable radially expansible braids are shown in a variety of patents, including U.S. Patent No. 6,080,174 and 7, 331,980, the disclosures of which are referred to.

Referring now to Fig. 9, a guidewire 160 comprises a guidewire body 162 having self-expanding anchor members 164 and a deployable blade 168. The anchor members 164 are disposed on opposite sides of the guidewire body and have pre-shaped generally spiral structures. An external sheath 170 may be axially advanced and retracted to close and to deploy the anchors 164. A deployable blade structure 168 is pivotally attached to be opened and closed within a slot 172 of the guidewire body 162. Tethers 174 are provided to open and close the blade. Such blade mechanisms are described in more detail in co-pending provisional application 61/171,228 (Attorney Docket No. 026923-001200US), the disclosure of which is referred to.

Referring now to Fig. 10, a guidewire 180 having a guidewire body 182 includes self-expanding ribbon anchors 184 which assume the illustrated shape when unconstrained and which may be collapsed into slots 186 (only one of which is illustrated) in the guidewire body 182 to allow the anchors to lie flat. They may be constrained by pulling on proximal ends of the anchors with tethers or using a constraining sheath. Cutting blade 190 is pivotally attached to move in and out of a second slot 192, where the slots 186 and 192 are orthogonally opposed (lie at 90 degrees relative to each other) so that the anchors and blades may be deployed without interference. Guidewire 180 is shown with a self-penetrating tip 196 but could also have non-penetrating tips.

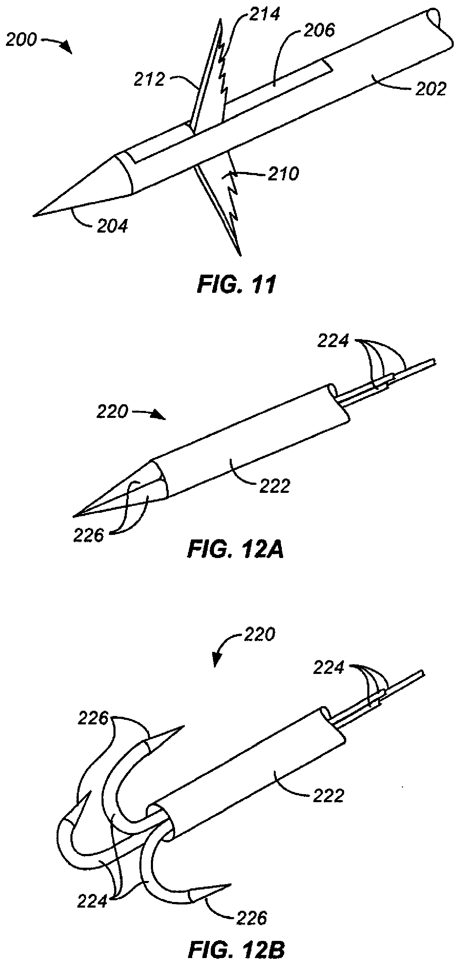

Referring now to Fig. 11, a guidewire 200 includes a guidewire body 202 having a self-penetrating tip 204 at its distal end. A single slot 206 is formed in the body and a single deployable structure 210 is provided which can be rotated in and out of the slot. Rotation can be achieved using tethers, springs, or the like. The structure 210 serves as both a cutting blade and as an anchor. A distal or forward edge 212 of the structure is sharpened so that it may cut tissue as the guidewire is advanced. The proximal side of the structure 214 is configured to grip tissue as the guidewire is drawn proximally into the tissue.

Referring now to Figs. 12A and 12B, a guidewire 220 comprises a tubular guidewire body 222 having three axially extendable elements 224 therein. Each of the elements 224 has a tissue-penetrating tip 226 where the tips will be drawn together to form a unitary tissue-penetrating tip, as shown in Fig. 12A, for passing through the tissue layers to form the initial tissue penetration. After the guidewire 220 has been advanced into the target body lumen, however, the elements 224 may be axially advanced through the body 222 so that the tips, which are preformed to evert as shown in Fig. 12B, will turn backwards and engage the tissue as an anchor. Such an anchor may be retracted by proximally drawing the elements 224 relative to the guidewire body 22.

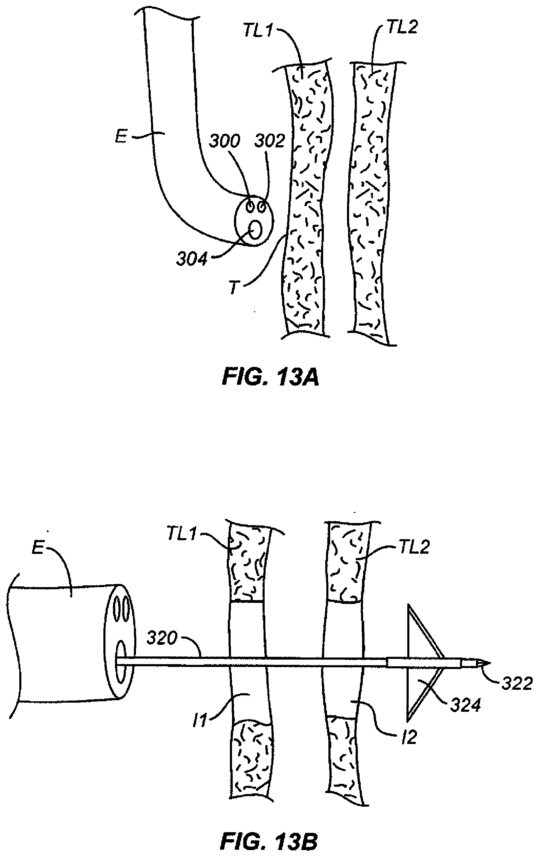

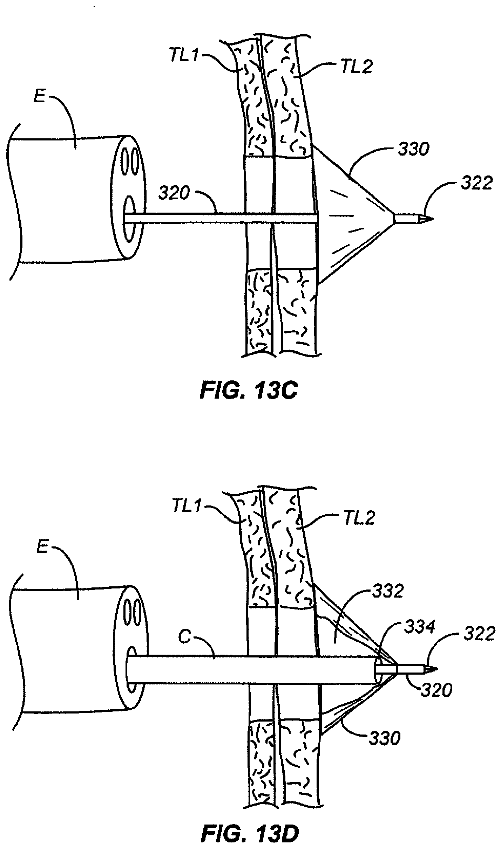

Referring now to Figs. 13A-13D, the methods according to the present disclosure will be described in more detail. As shown in Fig. 13A, an endoscope E may be advanced into an internal body space, such as the esophagus, to identify a target location T on a first tissue layer TL1. For example, the endoscope may include a viewing element 300 (typically an optical fiber or small camera) and an illuminating source 302 (typically an optical fiber or LED) to permit such visualization. The endoscope will also usually include a working channel 304 which may be used to advance a guidewire 320 in accordance with the principles of the present disclosure (Fig. 13B). Optionally, although not shown, a tissue penetration may be previously formed, for example by a trocar such as that described in co-pending application 61/171,228 (Attorney Docket No. 026923-001200US), the disclosure of which is referred to. As shown in Fig. 13B, however, the guidewire 320 in this example has a self-penetrating tip 322 and a penetration-enlarging deployable blade 324 so that advancement of the guidewire through tissue layers TL1 and TL2 provides lengthened incisions I1 and 12. Penetration of the guidewire 320 through the incisions I1 and I2 does not in itself draw the tissue layers TL1 and TL2 together as is desired. To achieve the desired tissue apposition, the blade 324 is retracted and a conical anchor 330 is deployed, as shown in Fig. 13C. The guidewire 320 is drawn proximally so that the deployed anchor 330 engages the second tissue layer TL2 and draws that layer against the first tissue layer TL1 to form a tight apposition, as shown in Fig. 13C. While maintaining the apposition, a catheter C may be advanced through the working channel of the endoscope, over the guidewire, and through the incisions I1 and 12, as shown in Fig. 13D. The catheter may be used for a variety of purposes, including drainage, stent placement, or the like. The conical balloon anchor 330 provides a working space 332 which allows the distal end 334 of the catheter C to pass through the tissue layer incisions I1 and I2 and into the working space 332 without disturbing engagement of the anchor 330 with the tissue, thus allowing tissue apposition to be maintained.

While the above is a complete description of the preferred embodiments of the disclosure, various alternatives, modifications, and equivalents may be used. Therefore, the above description should not be taken as limiting the scope of the invention which is defined by the appended claims.

## Claims

1. An apparatus for establishing an access tract from a first body lumen to a second body lumen, comprising a guidewire (140) having a guidewire body (144) having an expansible anchor (142) at or near its distal end, wherein the anchor (142) is configured to be deployed within the second body lumen and to be pulled proximally to draw the wall (TL2) of the second body lumen against the wall (TL1) of the first body lumen, wherein the anchor (142) is symmetric and adapted to engage the wall (TL2) of the second body lumen around an interface or lip which is generally circular and concentric with the access tract, wherein the guidewire body (144) has a length in the range from 150 to 250 cm, and an outside diameter, at least at the distal end, in the range from 0.4 mm to 5 mm, wherein the guidewire body (144) comprises a hollow center, wherein the anchor (142) is disposed near the distal end of the guidewire body (144) and expandable from a contracted configuration, which conforms closely to the exterior of the guidewire body (144), to an expanded configuration, **characterized in that** the expanded configuration will have a width or diameter at least four times as the diameter of the guidewire body (144), and that the anchor (142) is a radially expanding braid.

2. The apparatus of claim 1, wherein the anchor (142) has a concavity on a side adjacent to the wall (TL2) of the second body lumen.

3. The apparatus of any of the previous claims, wherein the anchor (142) on the guidewire body (144) has a retracted configuration which conforms to an external surface of the guidewire body (144) and an expanded configuration that has a distal surface and a proximal surface.

4. The apparatus of claim 3, wherein the proximal surface has a peripheral edge and a concave space or region within the peripheral edge,

5. The apparatus of any of the previous claims, wherein the guidewire body (144) has a tissue-penetrating tip (148).

6. The apparatus of claim 5, wherein the tissue-penetrating tip (148) is an electrosurgical tip.

7. The apparatus of any of the previous claims, wherein the width or diameter in the expanded configuration is at least six times as wide as the diameter of the guidewire body (144).

8. An endoscope (E) comprising a viewing element (300), an illuminating source (302) to permit visualization, a working channel (304), and the apparatus of any one of claims 1 to 7, wherein the apparatus is movably disposable in the working channel (304) of the endoscope (E).

## Patentansprüche

1. Vorrichtung zum Herstellen eines Zugangstrakts von einem ersten Körperlumen zu einem zweiten Körperlumen, mit einem Führungsdraht (140), der einen Führungsdrahtkörper (144) mit einem expandierbaren Anker (142) an oder in der Nähe seines distalen Endes aufweist, wobei der Anker (142) dafür konfiguriert ist, innerhalb des zweiten Körperlumens entfaltet und proximal gezogen zu werden, um die Wand (TL2) des zweiten Körperlumens gegen die Wand (TL1) des ersten Körperlumens zu ziehen, wobei der Anker (142) symmetrisch und dazu eingerichtet ist, die Wand (TL2) des zweiten Körperlumens um eine allgemein kreisförmige und sich konzentrisch zum Zugangstrakt erstreckende Grenzfläche oder Lippe herum in Eingriff zu bringen, wobei der Führungsdrahtkörper (144) eine Länge im Bereich von 150 bis 250 cm und einen Außendurchmesser, zumindest am distalen Ende, im Bereich von 0,4 mm bis 5 mm aufweist, wobei der Führungsdrahtkörper (144) eine hohle Mitte aufweist, wobei der Anker (142) in der Nähe des distalen Endes des Führungsdrahtkörpers (144) angeordnet ist und von einer zusammengezogenen Konfiguration, die mit einer Außenseite des Führungsdrahtkörpers (144) eng übereinstimmt, in eine expandierte Konfiguration expandierbar ist,
**dadurch gekennzeichnet, dass**
die expandierte Konfiguration eine Breite oder einen Durchmesser aufweisen wird, die/der mindestens viermal so groß ist wie der Durchmesser des Führungsdrahtkörpers (144) ist, und
der Anker (142) ein sich radial ausdehnendes Geflecht ist.

2. Vorrichtung nach Anspruch 1, wobei der Anker (142) auf einer Seite in der Nähe der Wand (TL2) des zweiten Körperlumens eine Konkavität aufweist.

3. Vorrichtung nach einem der vorangehenden Ansprüche, wobei der Anker (142) auf dem Führungsdrahtkörper (144) eine zurückgezogene Konfiguration, die mit einer Außenfläche des Führungsdrahtkörpers (144) übereinstimmt, und eine expandierte Konfiguration mit einer distalen Oberfläche und einer proximalen Oberfläche aufweist.

4. Vorrichtung nach Anspruch 3, wobei die proximale Oberfläche eine Umfangskante und einen konkaven Raum oder Bereich innerhalb der Umfangskante aufweist,

5. Vorrichtung nach einem der vorangehenden Ansprüche, wobei der Führungsdrahtkörper (144) eine Gewebe durchdringende Spitze (148) aufweist,

6. Vorrichtung nach Anspruch 5, wobei die Gewebe durchdringende Spitze (148) eine elektrochirurgische Spitze ist,

7. Vorrichtung nach einem der vorangehenden Ansprüche, wobei die Breite oder der Durchmesser in der expandierten Konfiguration mindestens sechsmal so breit ist wie der Durchmesser des Führungsdrahtkörpers (144).

8. Endoskop (E) mit einem Betrachtungselement (300), einer Beleuchtungsquelle (302) zum Ermöglichen einer Visualisierung, einem Arbeitskanal (304) und der Vorrichtung nach einem der Ansprüche 1 bis 7, wobei die Vorrichtung beweglich im Arbeitskanal (304) des Endoskops (E) anordenbar ist.

## Revendications

1. Appareil pour établir une voie d'accès d'une première lumière corporelle à une deuxième lumière corporelle, comprenant un fil de guidage (140) ayant un corps de fil de guidage (144) ayant un ancrage expansible (142) au niveau de ou à proximité de son extrémité distale, dans lequel l'ancrage (142) est configuré pour être déployé dans la deuxième lumière corporelle et pour être tiré de manière proximale afin d'attirer la paroi (TL2) de la deuxième lumière corporelle contre la paroi (TL1) de la première lumière corporelle, dans lequel l'ancrage (142) est symétrique et adapté pour mettre en prise la paroi (TL2) de la deuxième lumière corporelle autour d'une interface ou lèvre qui est généralement circulaire et concentrique avec la voie d'accès, dans lequel le corps de fil de guidage (144) a une longueur dans la plage allant de 150 à 250 cm, et un diamètre extérieur, au moins au niveau de l'extrémité distale, dans la plage allant de 0,4 mm à 5 mm, dans lequel le corps de fil de guidage (144) comprend un centre creux, dans lequel l'ancrage (142) est disposé à proximité de l'extrémité distale du corps de fil de guidage (144) et expansible à partir d'une configuration contractée, qui se conforme étroitement à l'extérieur du corps de fil de guidage (144), à une configuration expansée, **caractérisé en ce que** la configuration expansée a une largeur ou diamètre représentant au moins quatre fois le diamètre du corps de fil de guidage (144), et **en ce que** l'ancrage (142) est une tresse radialement expansible.

2. Appareil selon la revendication 1, dans lequel l'ancrage (142) a une concavité sur un côté adjacent à la paroi (TL2) de la deuxième lumière corporelle.

3. Appareil selon l'une quelconque des revendications précédentes, dans lequel l'ancrage (142) sur le corps de fil de guidage (144) a une configuration rétractée qui se conforme à une surface externe du corps de fil de guidage (144) et une configuration expansée qui a une surface distale et une surface proximale.

4. Appareil selon la revendication 3, dans lequel la surface proximale a un bord périphérique et un espace ou région concave à l'intérieur du bord périphérique.

5. Appareil selon l'une quelconque des revendications précédentes, dans lequel le corps de fil de guidage (144) a une pointe de pénétration de tissu (148).

6. Appareil selon la revendication 5, dans lequel la pointe de pénétration de tissu (148) est une pointe électrochirurgicale.

7. Appareil selon l'une quelconque des revendications précédentes, dans lequel la largeur ou diamètre, dans la configuration expansée, est au moins six fois plus large que le diamètre du corps de fil de guidage (144).

8. Endoscope (E) comprenant un élément de visualisation (300), une source d'éclairage (302) pour permettre la visualisation, un canal de travail (304) et l'appareil selon l'une quelconque des revendications 1 à 7, dans lequel l'appareil peut être disposé, de manière amovible, dans le canal de travail (304) de l'endoscope (E).
